# EUROPÄISCHE PATENTSCHRIFT

(11) **EP 2 746 384 B1**
(45) Veröffentlichungstag und Bekanntmachung des Hinweises auf die Patenterteilung: **27.07.2016**
(21) Anmeldenummer: 13198590.5
(22) Anmeldetag: 19.12.2013
(51) Int. Cl.: C12M 1/107, C12M 1/00, B65D 90/08, F17C 1/16

(54) **Klemmschienensystem**
Clamping rail system
Système de barres de serrage

(30) Priorität: 21.12.2012 DE 202012105047 U
(43) Veröffentlichungstag der Anmeldung: 25.06.2014
(73) Patentinhaber: JOPE Beteiligungs GmbH, 87653 Eggenthal (DE)
(72) Erfinder: Baur, Josef, 87784 Westerheim (DE); Baur, Peter, 87724 Ottobeuren (DE)
(74) Vertreter: Baumann, Rüdiger Walter

(56) Entgegenhaltungen:
- DE-A1-102011 106 755
- DE-U1-202005 003 358
- DE-U1-202005 011 689
- DE-U1-202012 101 425

## Beschreibung

Die Erfindung betrifft ein Klemmschienensystem für mindestens eine Folie insbesondere als Dach auf einem Fermenterbehälter einer Biogasanlage, sowie ein Schienenelement hierfür, nach dem Oberbegriff des Schutzanspruches 1.

Derartige gattungsgemäße Klemmschienensysteme nach dem bekannten Stand der Technik klemmen die Dachfolien entweder über Klemmschläuche in zugeordneten U-oder C- oder Schwalbenschwanzförmigen Schienen auf oder in einer insbesondere rohrzylindrischen Behälterwand ein, oder aber die Dachfolien sind zwischen zwei vertikal benachbarten auf der Oberseite der rohrzylindrischen Behälterwand aufgeschraubten Schienenketten eingeklemmt und gegebenenfalls formschlüssig festgelegt. DE202005011689U beschreibt eine Biogasanlage mit einem Klemmschienesystem. Nachteil der Klemmschläuche ist, dass diese nicht optimal gasdicht sind, weil die Klemmschläuche im Laufe der Zeit ihre Luft verlieren und dadurch die Klemmkraft nachlässt. Nachteil der Klemmschienen ist, dass diese kreisbogenförmig ausgestaltet sein müssen, da diese ja auf der Oberseite der rohrzylindrischen Behälterwand aufgeschraubt werden und dadurch eine Vielzahl von Krümmungsradien in abhängig vom Mittleren Durchmesser der rohrzylindrischen Behälterwand hergestellt, auf Lager gehalten und gegebenenfalls vor Ort auf die Baustelle mitgenommen werden müssen. Da diese Lagerhaltung sehr teuer ist, ist es auch bekannt, die Klemmleisten auftragsbezogen zu produzieren, was allerdings bei der Zeitplanung der Realisierung der Biogasanlage zu berücksichtigen ist, das heißt die Realisierungszeit verlängert sich. Zudem ist die Montage dadurch erschwert, dass der Monteur sehr präzise arbeiten muss, weil ansonsten eine leichte Abweichung von der kreiszylindrischen Form des Verlegens der Klemmschienen am Ende der Klemmschienenkette zu großen absoluten Fehlern führen würde und damit das Foliendach exzentrisch versetzt zur Behälterwand wäre oder gar das Foliendach nicht aufgesetzt werden könnte und das Klemmschienensystem erneut verlegt werden müsste. Dabei ist zu beachten, dass Montageungenauigkeiten hernach auch zu unerwünschten Undichtigkeiten der Biogasanlage führen können, da die mit dem Klemmschienensystem festgelegten Folien den gasproduzierenden Fermenterbehälter möglichst verlustfrei verschließen sollen, und Montageungenauigkeiten zu Spalten zwischen den einzelnen Schienenelementen führen, durch welche das gewonnene Gas zu entweichen droht.

Aufgabe der vorliegenden Erfindung ist es daher, ein gattungsgemäßes Klemmschienensystem derart weiter zu bilden, dass dieses kostengünstiger verfügbar ist.

Die gestellten Aufgaben werden durch die Merkmale des unabhängigen Anspruches 1 gelöst, wobei vorteilhafte Weiterbildungen Gegenstand der abhängigen Ansprüche sind.

Wesentliches Merkmal hierbei ist, dass die Schienenelemente im Wesentlichen gerade ausgebildet sind und am einen Ende eine konvexe Rundung und am anderen Ende eine hierzu zugeordnete komplementäre konkave Rundung besitzen, wobei die einander zugeordneten komplementären Rundungen horizontal benachbarter Schienenelemente derart ineinander greifen, dass eine relative Verschwenkung um einen vordefinierten Schwenkwinkel möglich ist.

Vorteil dabei ist, dass durch die Möglichkeit der einfachen Verschwenkung benachbarter Schienenelemente Positionierfehler beim Verlegen der Schienenelemente auf einem Kreis auf der Behälterwand leicht ausgeglichen werden können, so dass die ideale Kreisform durch einen Polygonzug innerhalb und/oder außerhalb der idealen Kreisform (Inkreis und/oder Umkreis) angenähert wird. Je kürzer die Schienenelemente in Relation zum Umfang bzw. Mittleren Durchmesser der Behälterwand sind, desto genauer kann man sich an die ideale Kreisform annähern. Je länger die Schienenelemente in Relation zum Umfang bzw. Mittleren Durchmesser der Behälterwand sind, desto kostengünstiger ist die Herstellung und Montage. Zudem wird durch die gerade Form anstatt der gebogenen Form der Schienenelemente deren Herstellung, Lagerhaltung und Montage kostengünstiger.

Natürlich können mit den erfindungsgemäßen Schienenelementen beliebige regelmäßige und/oder unregelmäßige Bogenformen einer Behälterwand abgedeckt werden, auch mit geraden Teilstücken, ja sogar bis hin zu rein polygonen Formen. Die Form der Behälterwand des Fermenterbehälters soll daher im Rahmen der vorliegenden Erfindung vollkommen frei bleiben.

Der erfindungsgemäße Vorschlag vereint günstige Lagerhaltung und hohe Flexibilität bzw. Verfügbarkeit des Klemmschienensystems bei der Realisierung eines entsprechenden Bauvorhabens. Die Schienenelemente des erfindungsgemäßen Klemmschienensystems werden in großen Losen kostengünstig produziert und stehen, weil diese dann auch mit einer vernünftigen Umschlagzahl auf Lager genommen werden können, kurzfristig zur Verfügung. Ergibt es sich, dass die Schienenkette nicht exakt zu einer Endloskette komplettierbar ist, wird einfach aus dem letzten Schienenelement der Überstand herausgeschnitten, wobei hier auf parallele Abschnittkanten geachtet wird, die dann möglichst spaltfrei zusammenfügbar sind. Dies kann problemlos auf der Baustelle erfolgen. Hieraus resultiert ein einfacher und zuverlässiger Einsatz des erfindungsgemäßen Vorschlags.

Insbesondere ist zwischen den horizontal benachbarten Schienenelementen kein oder nur ein sehr geringer Spalt zwischen ca. 0 und 5 mm vorhanden, so dass eine möglichst satte und großflächige Auflage der Schienenelemente auf der Dachfolie gewährleistet ist, ohne dass die Dachfolie durch Unterbrechungen in der Kette der Schienenelemente beschädigt werden könnte und/- oder Undichtigkeiten zum Innenraum des Fermenterbehälters oder gar ein Löslösen der Klemmung der Dachfolie entstehen könnten.

Besonders bevorzugt wird, dass der Schwenkwinkel zwischen benachbarten Schienenelementen, in Abhängigkeit des Mittleren Durchmessers der Behälterwand, im Bereich zwischen ca. 1° und ca. 25° liegt, typisch bei ca. 3,6° für einen Behälter mit Mittlerem Durchmesser der Behälterwand von 25 m bei je 100 Stück Schienenelementen der unteren und oberen Schienenkette. Hierdurch kann entweder mit vielen Schienenelementen möglichst genau oder aber mit möglichst wenig Schienenelementen kostengünstig die Kreisform bzw. Bogenform der Behälterwand nachgezogen werden.

Eine vorteilhafte Ausbildungsform sieht vor, dass die Schienenelemente der oberen und die unteren Schienenketten mit ihren Rundungen gegensinnig zueinander auf der Oberseite der Behälterwand orientiert sind, wobei insbesondere die konvexen Rundungen der Schienenelemente der oberen Schienenkette auf den konkaven Rundungen der Schienenelemente der unteren Schienenkette liegen und umgekehrt. Auch damit ist wieder sicher gestellt, dass die Schienenketten großflächig ununterbrochen auf den Folien aufliegen und diese festklemmen. Eine gleichsinnige Orientierung der oberen und unteren Schienenketten würde dazu führen, dass sich keine optimale Auflage im Spalt zwischen den Schienenelementen ausbildet, mit der Gefahr der Beschädigung der Folie und/oder der Undichtigkeit oder dem Löslösen der Folie.

Insbesondere kann vorgesehen sein, dass horizontal benachbarte Schienenelemente der oberen Schienenkette mittels jeweils einem horizontalen Verbindungsstreifen über vertikale Verbindungselemente miteinander verbunden sind, insbesondere derart, dass die Mittelpunkte der konkaven und konvexen Rundungen aufeinander liegen. Die vertikalen Verbindungselemente können gleichzeitig auch Halteelemente für die Formschlussverbindung zur unteren Schienenkette und/oder zur Behälterwand sein. Durch die Verbindungsstreifen kann der Spalt zwischen allen horizontal benachbarten Schienenelementen auf einen vordefinierten Bereich eingestellt werden, so dass eine gerade Anzahl von Schienenelementen ohne weitere Längenanpassungen eingesetzt werden kann. Auch kann hierdurch die Verbindung der Schienenkette zur Behälterwand verbessert werden und bei Versagen der Verbindung eines Schienenelementes zur Behälterwand kann dieses durch den Verbindungsstreifen sicher gehalten werden, ohne dass es zu einem ruckartigen Abfall des Gasdruckes im Innenraum des Fermenterbehälters führt, sondern schlimmstenfalls lediglich zu einem schleichenden Gasverlust, der dann bei Tragluftdächern durch das Luftgebläse ausgeglichen und bei Gelegenheit dann durch Reparatur oder Austausch des defekten Schienenelementes beseitigt werden kann.

Bevorzugt sind die vertikal benachbarten Schienenelemente der oberen und unteren Schienenkette an ihren beiden Enden mittels in die Behälterwand eingetriebenen Bolzen oder Schrauben, die durch Bohrungen in den Schienenelementen hindurchgreifen, miteinander verbunden. Damit wird eine einfache zusätzliche sehr robuste Formschlussverbindung beider Schienenketten zueinander und zur Behälterwand sicher gestellt.

In einer Alternative oder als Zusatz zu den zuvor erwähnten Schrauben/Bolzen können die vertikal benachbarten Schienenelemente der oberen und die unteren Schienenkette mittels Schraubzwingen oder anderen Klemmpratzen miteinander verbunden sein, welche an der Behälterwand ihr Widerlager finden. Diese Variante wird bevorzugt, wenn ein Bohren und Verschrauben in die Behälterwand vermieden werden soll oder nicht möglich ist, wobei jedoch die Behälterwand hierfür stoffschlüssige oder nachträglich montierte Widerlager aufweisen muss. Auch wird diese Variante gewählt, wenn bereits irgendwelche Widerlager, zum Beispiel Klemmschienen oder Regenwasserablaufschienen, auf der Außenseite der Behälterwand vorhanden sind, also wenn eine Biogasanlage mit bereits vorhandener (gegebenenfalls defekter oder renovierungsbedürftiger) Folienbefestigung umgerüstet werden soll.

Für die gasdichte Abdichtung der Schienenelemente der unteren Schienenkette zur Oberseite der Behälterwand wird insbesondere über eine ringförmig aufgetragene bei Montage plastische nach Montage aushärtende Dichtmasse vorgesehen. Diese Dichtmasse kann sehr schnell und unkompliziert und relativ breit auf der Oberseite der Behälterwand aufgetragen werden, womit dann die untere Schienenkette eine breite Varianz in der radialen Positionierung erhält, so dass damit Montagekosten eingespart werden können.

Es ist von Vorteil, wenn mindestens die Schienenelemente der unteren Schienenkette voneinander beabstandete Bohrungen oder Durchbrüche aufweisen, durch welche dann die plastische Dichtmasse während der Montage auf der Oberseite der Behälterwand hindurchtritt und nach Aushärten eine formschlüssige Verbindung herstellen. Es würde im Grunde genommen eine einzige gegebenenfalls relativ große Ausnehmung reichen, jedoch können aber auch eine Vielzahl solcher Bohrungen oder Durchbrüche durch die Schienenelemente für den Durchtritt der Dichtmasse vorgesehen sein. Es kann aber alternativ auch auf derartige Bohrungen oder Durchbrüche verzichtet werden, wenn die formschlüssigen Sicherungen in Form der Schrauben oder Bolzen der Schienenelemente zur Behälterwand in Ihrer Belastbarkeit zuverlässig ausgelegt sind.

Auch in den Rändern der konkaven und/oder konvexen Rundungen der unteren Schienenelemente können Ausklinkungen vorgesehen sein, durch welche Dichtmasse während der Montage hindurchtritt. Dies ist besonders dann erwünscht, wenn der Spalt zwischen den beiden benachbarten Schienenelementen der unteren Schienenkette relativ klein ist und dadurch die relativ zähe Dichtmasse nicht oder nicht genügend in den Spalt nach vertikal oben hindurchtreten und dadurch kein Formschluss erzielt werden kann.

Zur kostengünstigen Herstellung und Lagerung sind die Schienenelemente der unteren und oberen Schienenkette im Wesentlichen identisch ausgebildet, können sich aber auch geringfügig unterscheiden.

Der axiale Abstand zwischen dem tiefsten Punkt der konvexen Rundung und dem höchsten Punkt der konkaven Rundung eines Schienenelementes entspricht in einer bevorzugten Ausführungsform etwa der effektiven Länge von n/4 Meter, also ca. 78,54 cm +/- 1%. Hierdurch kann eine gerade Anzahl von Schienenelementen auf der Oberseite der Behälterwand aufgelegt und montiert werden, ohne dass es weiterer Anpassungen in der Länge des ersten oder letzten zu verlegenden Schienenelementes bedarf. So werden zum Beispiel für eine Behälterwand mit Mittlerem Durchmesser von 20 m genau 80 Stück Schienenelemente für die obere Schienenkette und genau 80 Stück Schienenelemente für die untere Schienenkette benötigt, wobei keines der Schienenelemente verkürzt werden muss. Auf Grund der geraden Schienenelemente und der leicht uneinheitlichen Spalte zwischen den Schienenelementen wird dann kein genau kreisförmiger Schienenkettenzug, sondern ein leicht polygoner Schienenkettenzug entstehen. Bei bevorzugter effektiven Länge für die Schienenelemente von n/4 Metern, also ca. 78,54 cm, liegt daher die benötigte Stückzahl der Schienenelemente bei 4 mal Mittel-Durchmesser der Behälterwand und der Schwenkwinkel zwischen benachbarten Schienenelementen bei 90° durch Mittel-Durchmesser.

Zur Ermöglichung des Schwenkwinkels zwischen zwei horizontal benachbarten Schienenelementen bis zu 25° und mehr können folgende Merkmale einzeln oder in Kombination vorgesehen sein. Die konvexe Rundung ist als 180°-Halbkreis und die konkave Rundung weniger als ein Halbkreis ausgebildet, insbesondere als Teilkreis zwischen 90° und 135°. Das Schienenelement weist im Bereich der konkaven Rundung, eine sich in Richtung der konkaven Rundung geringfügig verminderte Breite auf, das heißt, verläuft endseitig leicht zugespitzt, zwischen 1° und 5° Spitzenwinkel. Das Schienenelement weist im Bereich der konvexen Rundung eine geringfügig verminderte Breite in Form einer Freistellung auf, in welche die Ecken der konkaven Rundung des benachbarten Schienenelements bei Verschwenkung eintauchen.

Die Schienenelemente sind symmetrisch zu ihrer Längsachse, können aber als Variante asymmetrisch ausgebildet sein, wobei die Asymmetrie insbesondere die konstruktiven Merkmale betrifft, welche für die Verschwenkung benachbarter Schienenelemente zuständig sind, nämlich der Teilkreis der konkaven Rundung, der Spitzenwinkel bei der konkaven Rundung und die Freistellung bei der konvexen Rundung.

In den Zeichnungen ist die Erfindung insbesondere in einem Ausführungsbeispiel schematisch dargestellt. Es zeigen:
- Figur 1: Draufsicht auf einen Teilausschnitt einer Behälterwand eines Fermenterbehälters mit unterer Schienenkette des erfindungsgemäßen Klemmschienensystems und noch nicht montierten Dachfolien;
- Figur 2: Draufsicht auf einen Teilausschnitt eines Fermenterbehälters mit erfindungsgemäßem Klemmschienensystem und Dachfolien;
- Figur 3: Vergrößerte Detaildarstellung eines Schnitts durch die Oberseite der Behälterwand des Fermenterbehälters nach Figur 2 im Bereich einer Durchgangsbohrung der erfindungsgemäßen Schienenelemente;
- Figur 4a: Erfindungsgemäßes unteres Schienenelement nach Figur 1;
- Figur 4b: Erfindungsgemäßes unteres Schienenelement nach Figur 2;
- Figur 5: Vergrößerte Detaildarstellung auf Figur 1 im Bereich zweier benachbarter unterer Schienenelemente.

In den Figuren sind gleiche oder einander entsprechende Elemente jeweils mit den gleichen Bezugszeichen bezeichnet und werden daher, sofern nicht zweckmäßig, nicht erneut beschrieben.

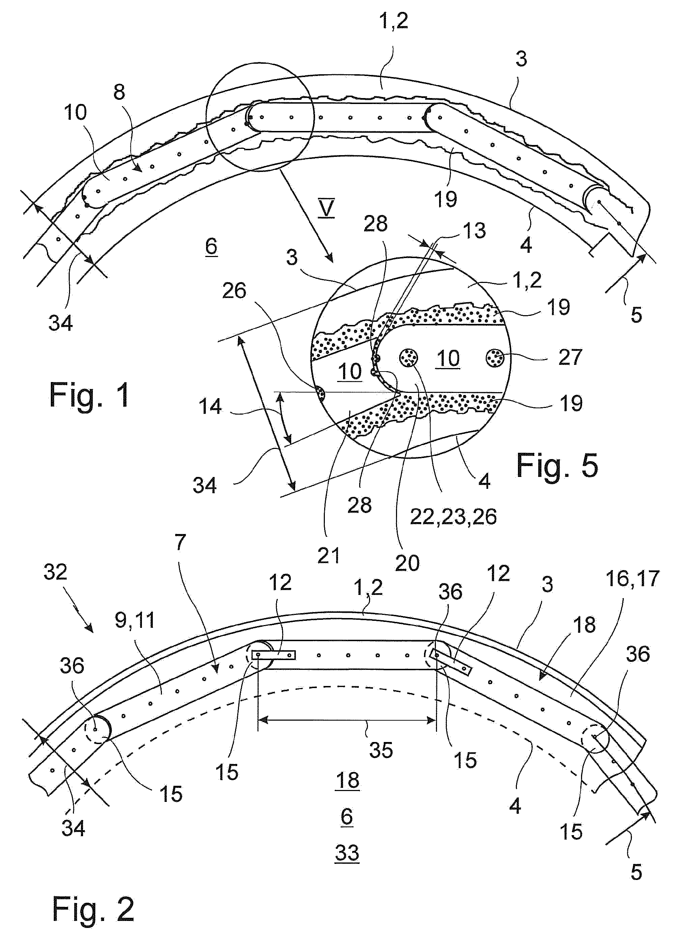

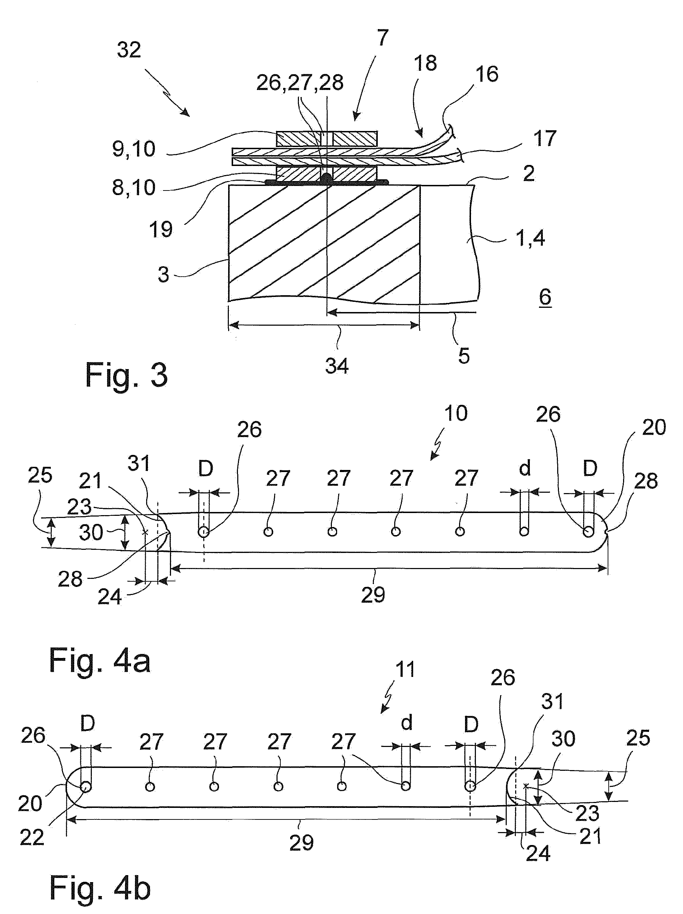

Figuren 2 und 3 stellen den fertigen Benutzungszustand eines Fermenterbehälters 32 für eine Biogasanlage dar.

Der Fermenterbehälter 32 wird definiert durch die kreiszylindrische Behälterwand 1, die Bodenplatte 33, das Foliendach 18, das Klemmschienensystem 7 und auch eine Dach-Stützkonstruktion (nicht dargestellt) und/oder ein Dach-Tragluftsystem (nicht dargestellt).

Die kreiszylindrische Behälterwand 1 ist zur Bodenplatte 33 hin flüssigkeitsdicht, sowie zum auf der Oberseite 2 der Behälterwand 1 mittels erfindungsgemäßem Klemmschienensystem 7 befestigen Foliendach 18 gasdicht (und ggf. flüssigkeitsdicht ausgebildet. Im hierdurch gas- und flüssigkeitsdichten Innenraum 6 des Fermenterbehälters 32 kann flüssige oder feste Silage zur Biogasgewinnung durch Fermentation (Gährung) gelagert werden. Das durch die Biogasfolie 17 des Foliendachs 18 im Innenraum 6 zurückgehaltene bzw. gespeicherte Biogas wird dann in an sich bekannter Weise abgesaugt und einer Wärme- und/oder Stromgewinnungsvorrichtung (zum Beispiel Verbrennungsmotor mit Kraft-/Wärmekopplung) der Biogasanlage zugeführt werden.

Oberhalb der Biogasfolie 17 des Foliendachs 18 befindet sich eine weitere Schutzfolie 16 des Foliendachs 18 als Schutz der Biogasfolie 17 vor mechanischer Beschädigung von außen. Beide übereinander liegenden Folien 16, 17 des Daches 18 sind etwa kegel- oder kuppelförmig mit einer etwa kreisrunden Basisfläche, so dass das Dach 18 am unteren freien Rand etwa kreisrund ausgebildet ist.

Wichtig ist nun, dass der etwa kreisrunde freie Rand beider Folien 16, 17 des Foliendachs 18 zwischen dem Klemmschienensystem 7 eingeklemmt ist, welches auf der Oberseite 2 des Behälters 1 festgelegt ist, so dass damit auch die beiden Folien 16, 17 am Behälter 1 gasdicht und gegebenenfalls flüssigkeitsdicht befestigt sind.

Das Klemmschienensystem 7 beinhaltet gemäß Figur 3 eine untere Schienenkette 8 mit einer Vielzahl aneinander seriell aufgereihten unteren Schienenelementen 10, welche in einer flüssigkeits- und gasdichtenden Dichtmasse 19 verlegt sind, wie am Besten in Figuren 1 und 5 erkennbar ist. Vertikal darüber liegt eine obere Schienenkette 9 mit einer Vielzahl aneinander seriell aufgereihten oberen Schienenelementen 11, wie sich aus Figur 2 am Besten ergibt. Zwischen den beiden Schienenketten 8 und 9 sind dann die beiden Folien 16, 17 des Daches 18 gasdicht und gegebenenfalls flüssigkeitsdicht eingeklemmt. Unterhalb der in Figur 2 dargestellten oberen Schienenkette 9 befindet sich daher die in der Figur 1 dargestellte untere Schienenkette 8, wobei sich zwischen den beiden Schienenketten 8, 9 die beiden Folien 16, 17 des Daches 18 befinden.

In Figur 5 ist gut zu erkennen, dass die horizontale Breite der Dichtmasse 19 größer ist, als die horizontale Breite der unteren Schienenelemente 10, so dass diese in einem relativ großen horizontalen Breitenbereich in ihrer Lage variabel verlegt werden können, wodurch es möglich wird, dass exakt eine ganze Anzahl von unteren Schienenelementen 10 (und damit auch oberen Schienenelementen 11) ohne weitere Kürzungsmaßnahmen eingesetzt werden können. Der durch die Schienenketten 8, 9 gebildete Ring auf der Oberseite 2 der Behälterwand 1 hat daher je nach gewählter effektiver Länge 29 der Schienenelemente 10, 11 und gewähltem Mittel-Durchmesser 5 der Behälterwand 1 einen größeren und/oder kleineren Durchmesser als der Mittel-Durchmesser 5 der Behälterwand 1. Die Schienenelemente 10, 11 sollten aber möglichst auf dem Mittel-Durchmesser 5 der Behälterwand 1 liegen, damit noch genügend Abstand zur die Dicke 34 definierenden Außenseite 3 und Innenseite 4 der Behälterwand 1 bleibt. Der Durchmesser der Schienenketten 8, 9 pendelt daher in der Regel etwas um den Mittel-Durchmesser 5 der Behälterwand 1 und ist mal größer, mal kleiner, so dass die Länge der Schienenketten 8, 9 etwa dem Umfang des Mittel-Durchmessers 5 der Behälterwand 1 entsprechen.

Hierzu werden jeweils benachbarte Schienenelemente 10, 11 der beiden Schienenketten 8, 9 zueinander nicht axial fluchtend, sondern im Winkel zueinander verschwenkt auf der Oberseite 2 der Behälterwand 1 angeordnet, so dass ein Schwenkwinkel 14 zwischen benachbarten Schienenelementen 10, 11 besteht, der im Bereich zwischen 1° und 25° liegen kann für Mittel-Durchmesser 5 der Behälterwand 1 zwischen 5 und 50 m. Insbesondere wird für die Schienenelemente 10, 11 eine bevorzugte effektiver Länge 29, gemessen zwischen dem tiefsten Punkt der konkaven Rundung 21 und dem höchsten Punkt der konvexen Rundung 20, von n/4 Metern gewählt, also ca. 78,54 cm, so dass dann die benötigte Stückzahl der Schienenelemente 10, 11 bei 4 mal Mittel-Durchmesser 5 der Behälterwand 1 und der Schwenkwinkel 14 bei 90° durch Mittel-Durchmesser 5 liegt.

Es werden bei zum Beispiel 5 m Mittel-Durchmesser 5 der Behälterwand 1 genau 20 Stück Schienenelemente 10, 11 je oben und unten benötigt, welche dann rechnerisch einen benachbarten Schwenkwinkel von 18° zueinander einnehmen, real vielleicht zwischen 16° und 18°. Bei zum Beispiel 10 m Mittel-Durchmesser 5 der Behälterwand 1 und 40 Stück Schienenelementen 10, 11 je oben und unten wäre der Schwenkwinkel bei rechnerisch 9°, real zwischen 8° und 10°.Bei zum Beispiel 25 m Mittel-Durchmesser 5 der Behälterwand 1 und 100 Stück Schienenelementen 10, 11 je oben und unten wäre der Schwenkwinkel bei rechnerisch 3,6°, real zwischen 3° und 4°. Bei zum Beispiel 50 m Mittel-Durchmesser 5 der Behälterwand 1 und 200 Stück Schienenelementen 10, 11 je oben und unten wäre der Schwenkwinkel bei rechnerisch 1,8°, real zwischen 1° und 2°.

Eine günstige Breite liegt für 78,54 cm (n/4 Meter) lange Schienenelemente 10, 11 bei ca. 7 cm, eine günstige Dicke bei ca. 0,5 cm.

In Figur 5 ist gut zu erkennen, dass der Spalt 13 zwischen benachbarten unteren Schienenelementen 10 sehr gering ist und etwa zwischen Null und 5 mm liegt. Beim Eindrücken der unteren Schienenelemente 10 in die pastöse Dichtmasse 19 quillt diese dann durch den Spalt 13 und wird durch die Durchbrüche 26, 27, 28 vertikal nach oben gedrückt und bildet nach Aushärtung einen Formschluss. Für den Fall, dass der Spalt 13 Null oder eben zu gering für das Hindurchtreten der Dichtmasse 19 sein sollte, sind Ausklinkungen 28 in den Rundungen 20, 21 der unteren Schienenelemente 10 für den Durchtritt der Dichtmasse 19 als Formschluss vorgesehen. Die Durchbrüche 26, 27, 28 sind auch für das Auffangen überschüssiger Dichtmasse 19 vorgesehen, damit die unteren Schienenelemente 10 einfach und schnell horizontal ausgerichtet und zum benachbarten unteren Schienenelement 10 vertikal fluchtend ausgerichtet werden können, ohne dass eine zu große Menge an Dichtmasse 19 über die Ränder der unteren Schienenelemente 10 heraus gedrückt werden muss.

In Figur 2 ist der Überlappungsbereich 15 der Rundungen 20, 21 der unteren 10 und oberen Schienenelemente 11 zu erkennen, wobei die konvexen Rundungen 20 der unteren Schienenelemente 10 vertikal oberhalb der konkaven Rundungen 21 der oberen Schienenelemente 11 liegen und analog umgekehrt, die konkaven Rundungen 21 der unteren Schienenelemente 10 vertikal oberhalb der konvexen Rundungen 20 der oberen Schienenelemente 11 liegen.

Die Verbindungsstreifen 12 zwischen benachbarten oberen Schienenelementen 11 dienen dazu, den Spalt 13 dazwischen jeweils konstant zu halten, so dass eine ganze Anzahl der oberen Schienenelemente 11 genutzt werden kann, ohne weitere Anpassungsmaßnahmen wie zum Beispiel Verkürzen oder Verlängern der oberen Schienenelemente 11. Damit ist natürlich auch eine ganze Anzahl der unteren Schienenelemente 10 sicher gestellt. Die Verbindungsstreifen 12 können gleichzeitig auch als vertikale Verbindungselemente zwischen den beiden Schienenketten 8, 9 dienen. Die Verbindungsstreifen 12 können gleichzeitig auch als Halteelemente für das Befestigen der Schienenketten 8, 9 auf der Oberseite 2 der Behälterwand 1 dienen.

Bevorzugt ist der Verbindungsstreifen 12 derart ausgebildet, dass dieser zwei Durchgangsbohrungen aufweist, in welche je eine Schraube (nicht dargestellt) hindurchgreift und die Schienenketten 8, 9 auf der Oberseite 2 der Behälterwand 1 formschlüssig festlegen. Eine der beiden Bohrungen des Verbindungsstreifens 12 wird auf dem Mittelpunkt 22 der konvexen Rundung 20 eines Schienenelementes 10, 11 aufgelegt, die andere Bohrung des Verbindungsstreifens 12 befindet sich dann automatisch derart vom Mittelpunkt 23 der Konkaven Rundung 21 des benachbarten Schienenelementes 10, 11 entfernt, dass eine Verschwenkung der beiden benachbarten Schienenelemente 10, 11 beider Schienenketten 8, 9 um den vordefinierten Schwenkwinkel 14 möglich ist.

Schließlich sind in den Figuren 4a und 4b das untere 10 und obere Schienenelement 11 näher dargestellt, wobei die beiden Schienenelemente 10 und 11 in sich achssymmetrisch aufgebaut sind, sowie zueinander nahezu identisch aufgebaut sind, mit Ausnahme der randseitigen Ausklinkungen 28 in den Rundungen 20, 21 auf der Längsachse des unteren Schienenelements 10.

Es sind jeweils die endseitigen durchmessergrößeren Bohrungen 26 für die Halteschrauben (nicht gezeigt) der Verbindungsstreifen 12 vorhanden, sowie dazwischen befindliche gleich beabstandete durchmesserkleinere Bohrungen 27 für den Formschluss mittels der ausgehärteten Dichtmasse 19, wie in Figur 3 angedeutet ist.

Mit dem bloßen Auge kaum zu erkennen ist hingegen die endseitige Anspitzung mit Spitzenwinkel 25 zwischen 1° und 5° im Bereich der konkaven Rundungen 21 beider Schienenelemente 10, 11. Durch den Spitzenwinkel 25 ergibt sich eine gegenüber der Normalbreite von zum Beispiel 70 mm verminderte Breite 30 von zum Beispiel 65 mm im Endbereich. Die konkaven Rundungen 21 sind hierbei um die axiale Kürzung 24 der Schienenelemente 10, 11 verkürzt und daher nicht wie die konvexen Rundungen 20 als 180°-Halbkreis ausgebildet, sondern lediglich als Teilkreis im Bereich zwischen 90° und 135°. Durch beide konstruktive Maßnahmen, der Kürzung 24 und den Spitzenwinkel 25, wird der Verschwenkwinkel 14 zwischen benachbarten Schienenelementen 10, 11 zwischen 1° und 25° erst ermöglicht. Die beiden Spitzen 31 im Bereich der konkaven Rundung 20 sind abgerundet, damit die Folien 16, 17 keinen Schaden nehmen.

Die axiale effektive Länge 29 auf der Längsachse der Schienenelemente 10, 11 zwischen den beiden Rundungen 20, 21 der Schienenelemente 10, 11 liegt bevorzugt bei n/4 Metern, also bei 78,54 cm, wodurch eine ganze Anzahl von Schienenelementen 10, 11 beider Schienenketten 8, 9 möglich wird. Diese axiale effektive Länge 29 entspricht dem Abstand 35 zwischen zwei Drehpunkten 36 der Schienenketten 8, 9, wobei die Drehpunkte 36 den Mittelpunkten 22, 23 der Rundungen 20, 21 entsprechen und zur Bildung des Schwenkwinkels 14 dienen.

Insgesamt wird damit ein gattungsgemäßes Klemmschienensystem bereit gestellt, das sehr einfach, schnell, zuverlässig und kostengünstig herstellbar und montierbar ist.

Der Vorschlag umfasst:
Ein Klemmschienensystem (7) für mindestens eine Folie (16, 17) insbesondere als Dach (18) auf einem Fermentationsbehälter einer Biogasanlage, mit einer, einen Behälterinnenraum (6) umgebenden, insbesondere etwa rohrzylinderförmigen Behälterwand (1), auf dessen Oberseite (2) das Klemmschienensystem (7) festgelegt ist, welches eine untere Schienenkette (8) und eine hierzu vertikal darüber liegende obere Schienenkette (9) mit jeweils einer Vielzahl von Schienenelementen (10, 11) beinhaltet, wobei zwischen unterer (8) und oberer Schienenkette (9) die mindestens eine Folie (16, 17) kraftschlüssig eingeklemmt und dort gegebenenfalls formschlüssig mittels Halteelementen festgelegt ist, wobei die Schienenelemente (10, 11) im Wesentlichen gerade ausgebildet sind und am einen Ende eine konvexe Rundung (20) und am anderen Ende eine hierzu zugeordnete komplementäre konkave Rundung (21) besitzen, wobei die einander zugeordneten komplementären Rundungen (20, 21) horizontal benachbarter Schienenelemente (10; 11) derart ineinander greifen, dass eine relative Verschwenkung um einen Schwenkwinkel (14) möglich ist.

Ein wie zuvor ausgeführtes Klemmschienensystem, wobei zwischen horizontal benachbarten Schienenelementen (10; 11) kein oder nur ein sehr geringer Spalt (13) zwischen ca. 0 und 5 mm vorhanden ist.

Ein wie zuvor ausgeführtes Klemmschienensystem, wobei der Schwenkwinkel (14), in Abhängigkeit des Mittleren Durchmessers (5) der rohrzylindrischen Behälterwand (1) und der axialen effektiven Länge (29) des Schienenelementes (10, 11), im Bereich zwischen ca. 1° und ca. 25° liegt, und insbesondere der reale Schwenkwinkel (14) in Abhängigkeit der Dicke (34) der Behälterwand (1) eine Abweichung des rechnerischen Schwenkwinkels (14) von +/- 1% bis 10% aufweist.

Ein wie zuvor ausgeführtes Klemmschienensystem, wobei die Schienenelemente (10, 11) der oberen und die unteren Schienenketten (8, 9) mit ihren Rundungen (20, 21) gegensinnig zueinander auf der Oberseite der Behälterwand (1) orientiert sind.

Ein wie zuvor ausgeführtes Klemmschienensystem, wobei die Rundungen (20, 21) der Schienenelemente (10, 11) der oberen und unteren Schienenketten (8, 9) jeweils vertikal übereinander liegen.

Ein wie zuvor ausgeführtes Klemmschienensystem, wobei die konvexen Rundungen (20) der Schienenelemente (11) der oberen Schienenkette (9) vertikal über den konkaven Rundungen (21) der Schienenelemente (10) der unteren Schienenkette (8) liegen und umgekehrt.

Ein wie zuvor ausgeführtes Klemmschienensystem, wobei horizontal benachbarte obere Schienenelemente (11) der oberen Schienenkette (9) mittels horizontaler Verbindungsstreifen (12) und vertikalen Verbindungselementen miteinander verbunden sind.

Ein wie zuvor ausgeführtes Klemmschienensystem, wobei die Verbindungselemente der Verbindungsstreifen (12) zugleich Halteelemente zur Formschlussverbindung zur Oberseite (2) der Behälterwand (1) sind.

Ein wie zuvor ausgeführtes Klemmschienensystem, wobei die Mittelpunkte (22, 23) einander zugeordneter konvexen (20) und konkaven Rundungen (21) benachbarter oberer Schienenelemente (11) etwa aufeinander liegen.

Ein wie zuvor ausgeführtes Klemmschienensystem, wobei vertikal benachbarte Schienenelemente (10, 11) der oberen (9) und unteren Schienenkette (8) mittels vertikaler Halteelemente miteinander verbunden sind.

Ein wie zuvor ausgeführtes Klemmschienensystem, wobei vertikal benachbarte Schienenelemente (10, 11) der oberen (9) und unteren Schienenkette (8) an ihren beiden Enden mittels der vertikalen Halteelemente in Form von Bolzen oder Schrauben, die durch Durchbrechungen oder Bohrungen (26) in den Schienenelementen (10, 11) hindurchgreifen, miteinander verbunden sind, wobei die Bolzen oder Schrauben in die Behälterwand (1) eingetrieben sind.

Ein wie zuvor ausgeführtes Klemmschienensystem, wobei die Schienenelemente (10) der unteren Schienenkette (8) zur Oberseite (2) der Behälterwand (1) insbesondere über eine ringförmig aufgetragene, bei Montage plastische, nach Montage aushärtende, Dichtmasse (19) gasdicht abgedichtet ist.

Ein wie zuvor ausgeführtes Klemmschienensystem, wobei mindestens die Schienenelemente (10) der unteren Schienenkette (8) voneinander beabstandete Bohrungen (27) oder Durchbrüche aufweisen, durch welche die plastische Dichtmasse (19) während der Montage auf der Oberseite (2) der Behälterwand (1) hindurchtritt und nach Aushärten eine formschlüssige Verbindung herstellt.

Ein wie zuvor ausgeführtes Klemmschienensystem, wobei in den Rändern der konkaven und/oder konvexen Rundungen (20, 21) der unteren Schienenelemente (10) Ausklinkungen (28) vorgesehen sind, durch welche Dichtmasse (19) während der Montage auf der Oberseite (2) der Behälterwand (1) hindurchtritt und nach Aushärten eine formschlüssige und/oder gasdichte Verbindung herstellt.

Ein wie zuvor ausgeführtes Klemmschienensystem, wobei alle oder fast alle die Schienenelemente (10, 11) der unteren (8) und oberen Schienenkette (9) im Wesentlichen identisch ausgebildet sind.

Der Vorschlag umfasst auch ein Schienenelement für ein wie zuvor beschriebenes Klemmschienensystem, wobei der axiale Abstand (29) zwischen dem tiefsten Punkt der konvexen Rundung (20) und dem höchsten Punkt der konkaven Rundung (21) eines Schienenelementes (10, 11) etwa eine effektive Länge (29) von n/4 Meter, also ca. 78,54 +/- 1% cm, aufweist.

Ein wie zuvor beschriebenes Schienenelement, wobei die konvexe Rundung (20) als 180°-Halbkreis und die konkave Rundung (21) weniger als ein Halbkreis, insbesondere als Teilkreis zwischen ca. 90° und 135° ausgebildet ist.

Ein wie zuvor beschriebenes Schienenelement, wobei das Schienenelement (10, 11) im Bereich der konkaven Rundung (21), eine sich in Richtung der konkaven Rundung (21) geringfügig verminderte Breite (30) aufweist, das heißt, endseitig leicht zugespitzt verläuft, mit einem Spitzenwinkel (25) zwischen ca. 1° und 5°.

Ein wie zuvor beschriebenes Schienenelement, wobei das Schienenelement (10, 11) im Bereich der konvexen Rundung (20) eine geringfügig verminderte Breite (30) aufweist, in welche die Ecken der konkaven Rundung (21) des horizontal benachbarten Schienenelements (10; 11) bei Verschwenkung um den Schwenkwinkel (14) eintauchen.

Die im Zusammenhang mit den einzelnen Ausführungsformen des Vorschlags beschriebenen Merkmale, Teilmerkmale und/oder Merkmalskombinationen können selbstverständlich beliebig kombiniert werden, um den erfindungsgemäßen Gegenstand vorteilhaft weiterzubilden. Auch die Kombinationen von Merkmalen, Teilmerkmalen und/oder Merkmalskombinationen aus Ansprüchen verschiedener Anspruchskategorien sind gleichermaßen umfasst.

Die jetzt mit der Anmeldung und später eingereichten Ansprüche sind Versuche zur Formulierung ohne Präjudiz für die Erzielung weitergehenden Schutzes.

Sollte sich hier bei näherer Prüfung, insbesondere auch des einschlägigen Standes der Technik, ergeben, daß das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist.

Es ist weiter zu beachten, daß die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Merkmale, die bislang nur in der Beschreibung offenbart wurden, können im Laufe des Verfahrens als von erfindungswesentlicher Bedeutung, zum Beispiel zur Abgrenzung vom Stand der Technik beansprucht werden.

Merkmale, die nur in der Beschreibung offenbart wurden, oder auch Einzelmerkmale aus Ansprüchen, die eine Mehrzahl von Merkmalen umfassen, können jederzeit zur Abgrenzung vom Stande der Technik in den ersten Anspruch übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

## Patentansprüche

1. Klemmschienensystem (7) für mindestens eine Folie (16, 17) insbesondere als Dach (18) auf einem Fermentationsbehälter einer Biogasanlage, mit einer, einen Behälterinnenraum (6) umgebenden, insbesondere etwa rohrzylinderförmigen Behälterwand (1), auf dessen Oberseite (2) das Klemmschienensystem (7) festgelegt ist, welches eine untere Schienenkette (8) und eine hierzu vertikal darüber liegende obere Schienenkette (9) mit jeweils einer Vielzahl von Schienenelementen (10, 11) beinhaltet, wobei zwischen unterer (8) und oberer Schienenkette (9) die mindestens eine Folie (16, 17) kraftschlüssig eingeklemmt und dort gegebenenfalls formschlüssig mittels Halteelementen festgelegt ist, **dadurch gekennzeichnet, dass** die Schienenelemente (10, 11) im Wesentlichen gerade ausgebildet sind und am einen Ende eine konvexe Rundung (20) und am anderen Ende eine hierzu zugeordnete komplementäre konkave Rundung (21) besitzen, wobei die einander zugeordneten komplementären Rundungen (20, 21) horizontal benachbarter Schienenelemente (10; 11) derart ineinander greifen, dass eine relative Verschwenkung um einen Schwenkwinkel (14) möglich ist.

2. Klemmschienensystem nach Anspruch 1, **dadurch gekennzeichnet, dass** zwischen horizontal benachbarten Schienenelementen (10; 11) kein oder nur ein sehr geringer Spalt (13) zwischen ca. 0 und 5 mm vorhanden ist.

3. Klemmschienensystem nach Anspruch 1 oder 2, **dadurch gekennzeichnet, dass** der Schwenkwinkel (14), in Abhängigkeit des Mittleren Durchmessers (5) der rohrzylindrischen Behälterwand (1) und der axialen effektiven Länge (29) des Schienenelementes (10, 11), im Bereich zwischen ca. 1° und ca. 25° liegt, und insbesondere der reale Schwenkwinkel (14) in Abhängigkeit der Dicke (34) der Behälterwand (1) eine Abweichung des rechnerischen Schwenkwinkels (14) von +/- 1% bis 10% aufweist.

4. Klemmschienensystem nach einem der vorhergehenden Ansprüche, **dadurch gekennzeichnet, dass** die Schienenelemente (10, 11) der oberen und die unteren Schienenketten (8, 9) mit ihren Rundungen (20, 21) gegensinnig zueinander auf der Oberseite der Behälterwand (1) orientiert sind.

5. Klemmschienensystem nach einem der vorhergehenden Ansprüche, **dadurch gekennzeichnet, dass** die Rundungen (20, 21) der Schienenelemente (10, 11) der oberen und unteren Schienenketten (8, 9) jeweils vertikal übereinander liegen.

6. Klemmschienensystem nach einem der vorhergehenden Ansprüche, **dadurch gekennzeichnet, dass** die konvexen Rundungen (20) der Schienenelemente (11) der oberen Schienenkette (9) vertikal über den konkaven Rundungen (21) der Schienenelemente (10) der unteren Schienenkette (8) liegen und umgekehrt.

7. Klemmschienensystem nach einem der vorhergehenden Ansprüche, **dadurch gekennzeichnet, dass** horizontal benachbarte obere Schienenelemente (11) der oberen Schienenkette (9) mittels horizontaler Verbindungsstreifen (12) und vertikalen Verbindungselementen miteinander verbunden sind, insbesondere wobei die Verbindungselemente der Verbindungsstreifen (12) zugleich Halteelemente zur Formschlussverbindung zur Oberseite (2) der Behälterwand (1) sind und/oder dadurch, dass die Mittelpunkte (22, 23) einander zugeordneter konvexen (20) und konkaven Rundungen (21) benachbarter oberer Schienenelemente (11) etwa aufeinander liegen.

8. Klemmschienensystem nach einem der vorhergehenden Ansprüche, **dadurch gekennzeichnet, dass** vertikal benachbarte Schienenelemente (10, 11) der oberen (9) und unteren Schienenkette (8) mittels vertikaler Halteelemente miteinander verbunden sind.

9. Klemmschienensystem nach Anspruch 8, **dadurch gekennzeichnet, dass** vertikal benachbarte Schienenelemente (10, 11) der oberen (9) und unteren Schienenkette (8) an ihren beiden Enden mittels der vertikalen Halteelemente in Form von Bolzen oder Schrauben, die durch Durchbrechungen oder Bohrungen (26) in den Schienenelementen (10, 11) hindurchgreifen, miteinander verbunden sind, wobei die Bolzen oder Schrauben in die Behälterwand (1) eingetrieben sind.

10. Klemmschienensystem nach einem oder mehreren der vorhergehenden Ansprüche, **dadurch gekennzeichnet, dass** die Schienenelemente (10) der unteren Schienenkette (8) zur Oberseite (2) der Behälterwand (1) insbesondere über eine ringförmig aufgetragene, bei Montage plastische, nach Montage aushärtende, Dichtmasse (19) gasdicht abgedichtet ist und/oder dadurch, dass mindestens die Schienenelemente (10) der unteren Schienenkette (8) voneinander beabstandete Bohrungen (27) oder Durchbrüche aufweisen, durch welche die plastische Dichtmasse (19) während der Montage auf der Oberseite (2) der Behälterwand (1) hindurchtritt und nach Aushärten eine formschlüssige Verbindung herstellt.

11. Klemmschienensystem nach Anspruch 10, **dadurch gekennzeichnet, dass** in den Rändern der konkaven und/oder konvexen Rundungen (20, 21) der unteren Schienenelemente (10) Ausklinkungen (28) vorgesehen sind, durch welche Dichtmasse (19) während der Montage auf der Oberseite (2) der Behälterwand (1) hindurchtritt und nach Aushärten eine formschlüssige und/oder gasdichte Verbindung herstellt.

12. Klemmschienensystem nach einem der vorhergehenden Ansprüche, **dadurch gekennzeichnet, dass** alle oder fast alle die Schienenelemente (10, 11) der unteren (8) und oberen Schienenkette (9) im Wesentlichen identisch ausgebildet sind.

13. Schienenelement für ein Klemmschienensystem nach einem der vorhergehenden Ansprüche, **dadurch gekennzeichnet, dass** der axiale Abstand (29) zwischen dem tiefsten Punkt der konvexen Rundung (20) und dem höchsten Punkt der konkaven Rundung (21) eines Schienenelementes (10, 11) etwa eine effektive Länge (29) von n/4 Meter, also ca. 78,54 +/- 1% cm, aufweist.

14. Schienenelement nach Anspruch 13, **dadurch gekennzeichnet, dass** die konvexe Rundung (20) als 180°-Halbkreis und die konkave Rundung (21) weniger als ein Halbkreis, insbesondere als Teilkreis zwischen ca. 90° und 135° ausgebildet ist und/oder dadurch, dass das Schienenelement (10, 11) im Bereich der konkaven Rundung (21), eine sich in Richtung der konkaven Rundung (21) geringfügig verminderte Breite (30) aufweist, das heißt, endseitig leicht zugespitzt verläuft, mit einem Spitzenwinkel (25) zwischen ca. 1° und 5°.

15. Schienenelement für ein Klemmschienensystem nach Anspruch 13 oder 14, **dadurch gekennzeichnet, dass** das Schienenelement (10, 11) im Bereich der konvexen Rundung (20) eine geringfügig verminderte Breite (30) aufweist, in welche die Ecken der konkaven Rundung (21) des horizontal benachbarten Schienenelements (10; 11) bei Verschwenkung um den Schwenkwinkel (14) eintauchen.

## Claims

1. Clamping rail system (7) for at least one film (16, 17), in particular as roof (18) on a fermentation tank of a biogas plant, with an about pipe cylinder-shaped tank wall (1) surrounding a tank interior (6), on the upper side (2) of the tank wall the clamping rail system (7) being fixed, that contains a bottom rail chain (8) and a top rail chain (9) arranged hereto vertically above it each having a multitude of rail elements (10, 11), wherein between bottom (8) and top (9) rail chain the at least one film (16, 17) is clamped force-fittingly, and there is also fixed form-fittingly by means of holding elements, **characterized in that** the rail elements (10, 11) are designed essentially straight, and have at one of its ends a convex curve (20) and at the other of its ends a concave curve (21) complementarily assigned hereto, wherein the assigned complementary curves (20, 21) of horizontally adjacent rail elements (10, 11) are engaged such that a relative pivoting about a pivot angle (14) is possible.

2. Clamping rail system according to claim 1, **characterized in that** between horizontally adjacent rail elements (10, 11) there is no or only a very slight gap (13) of between about 0 and 5 mm.

3. Clamping rail system according to claims 1 or 2, **characterized in that** the pivot angle (14), depending on the medium diameter (5) of the pipe cylindrical tank wall (1) and the axial effective length (29) of the rail element (10, 11), is in the range between about 1° and about 25°, and in particular the real pivot angle (14), depending on the thickness (34) of the tank wall (1) has a discrepancy of the calculated pivot angle (14) of +/- 1% to 10%.

4. Clamping rail system according to one of the preceding claims, **characterized in that** the rail elements (10, 11) of the top and bottom rail chains (8, 9) along with their curves (20, 21) are orientated in opposite direction to each other on the upper side of the tank wall (1).

5. Clamping rail system according to one of the preceding claims, **characterized in that** the curves (20, 21) of the rail elements (10, 11) of the top and bottom rail chains (8, 9) each are arranged vertically one above the other.

6. Clamping rail system according to one of the preceding claims, **characterized in that** the convex curves (20) of the rail elements (11) of the top rail chain (9) are arranged vertically above the concave curves (21) of the rail elements (10) of the bottom rail chain (8) and vice versa.

7. Clamping rail system according to one of the preceding claims, **characterized in that** horizontally adjacent top rail elements (11) of the top rail chain (9) are connected to one another by means horizontal connection strips (12) and vertical connection elements, in particular wherein the connection elements of the connection strips (12) are also holding elements for the form-fitting connection with the upper side (2) of the tank wall (1), and/or **in that** the center points (22, 23) of assigned convex (20) and concave (21) curves of adjacent top rail elements (11) rest about one upon the other.

8. Clamping rail system according to one of the preceding claims, **characterized in that** vertically adjacent rail elements (10, 11) of the top (9) and bottom (8) rail chain are connected to one another by means of vertical holding elements.

9. Clamping rail system according to claim 8, **characterized in that** vertically adjacent rail elements (10, 11) of the top (9) and bottom (8) rail chain are connected to each other on both of their ends by means of the vertical holding elements in form of bolts or screws penetrating breakthroughs or bore holes (26) in the rail elements (10, 11), the bolts or screws being hammered in the tank wall (1).

10. Clamping rail system according to one or more of the preceding claims, **characterized in that** the rail elements (10) of the bottom rail chain (8) are sealed gas-proof towards the upper side (2) of the tank wall (1), in particular through an annularly applied sealing compound, that is plastic during mounting and hardens after mounting, and/or that at least the rail elements (10) of the bottom rail chain (8) have spaced bore holes (27) or breakthroughs which the plastic sealing compound (19) penetrates during mounting on the upper side (2) of the tank wall (1), and produces a form-fitting connection after hardening.

11. Clamping rail system according to claim 10, **characterized in that** in the edges of the concave and/or convex curves (20, 21) of the bottom rail elements (10) notches (28) are provided which are penetrated by the sealing compound (19) during mounting on the upper side (2) of the tank wall (1), and after hardening produces a form-fitting and/or gas-proof connection.

12. Clamping rail system according to one of the preceding claims, **characterized in that** all or nearly all the rail elements (10, 11) of the bottom (8) and top (9) rail chain are configured essentially identically.

13. Rail element for a clamping rail system according to one of the preceding claims, **characterized in that** the axial distance (29) between the lowest point of the convex curve (20) and the highest point of the concave curve (21) of a rail element (10, 11) has roughly an effective length (29) of /4 meters, that is 78.54 cm +/- 1%.

14. Rail system according to claim 13, **characterized in that** the convex curve (20) is configured as 180° semi-circle, and the concave curve (21) is configured less than a semi-circle, in particular as part-circle between 90° and 135°, and/or **in that** the rail element (10, 11) has in the region of the concave curve (21) a width (30) that is slightly reduced in the direction of the concave curve (21), that means it is slightly pointed on the side of the end, with an acute angle (25) between 1° and 5°.

15. Rail element for a clamping rail system according to claim 13 or 14, **characterized in that** the rail element (10, 11) has a slightly reduced width (30) in the region of the convex curve (20) in which the corners of the concave curve (21) of the horizontally adjacent rail element (10, 11) immerse when pivoted about the pivot angle (14).

## Revendications

1. Système de barres de serrage (7) destiné à au moins un film (16, 17), en particulier comme toit (18) au-dessus d'un conteneur de fermentation d'une installation biogaz, comprenant une paroi de conteneur (1) fermant un espace intérieur (6) du conteneur et ayant en particulier une forme approchant celle d'un tube cylindrique, avec, fixé à sa face supérieure, le système de barres de serrage (7) comportant une chaîne de barres inférieures (8) et, située verticalement au-dessus, une chaine de barres supérieures (9), les deux chaînes contenant de nombreux éléments de barres (10, 11) et le ou les film(s) (16, 17) serré(s) et maintenu(s) par frottement et, le cas échéant par des éléments de fixation entre les chaînes de barres inférieures (8) et supérieures (9), **caractérisé en ce que** les éléments de barre(10, 11) ont une forme essentiellement rectiligne et possèdentà une extrémité un arrondi convexe (20) et à l'autre extrémité un arrondi concave (21) correspondant de façon à ce que les arrondis complémentaires (20, 21) et correspondant aux deux éléments de barre horizontalement voisins interagissent de façon à permettre le pivotement sous un angle de pivotement (14).

2. Système de barres de serrage selon la revendication 1, **caractérisé en ce que** la fente (13) entre des éléments de barre horizontalement voisins (10, 11) est inexistante ou très petite, variant entre 0 mm et 5 mm environ.

3. Système de barres de serrage selon la revendication 1 ou 2, **caractérisé en ce que** l'angle de pivotement (14) se situe entre 1° et 25°environ en fonction du diamètre moyen (5) de la paroi de conteneur (1) en forme d'un tube cylindrique et de la longueur axiale effective (29) de l'élément de barre (10, 11), et **en ce qu'**en particulier l'angle de pivotement (14) réel comporte une différence par rapport à l'angle de pivotement (14) calculé de +/- 1% jusqu'à 10% en fonction de l'épaisseur (34) de la paroi de conteneur (1).

4. Système de barres de serrage selon une des revendications précédentes, **caractérisé en ce que** les éléments de barre (10, 11) de la chaîne de barres supérieure et inférieure (8, 9) sont orientés par rapport à leurs arrondis en sens opposés au niveau de la partie supérieure de la paroi de conteneur (1).

5. Système de barres de serrage selon une des revendications précédentes, **caractérisé en ce que** les arrondis (20, 21) des éléments de barre (10, 11) des chaînes de barres supérieures et inférieures (8, 9) sont situés verticalement l'un au-dessus de l'autre.

6. Système de barres de serrage selon une des revendications précédentes, **caractérisé en ce que** les arrondis convexes (20) des éléments de barre (11) de la chaîne de barres supérieure sont situés verticalement au-dessus des arrondis concaves (21) des éléments de barre (10) de la chaîne de barres inférieure (8) et réciproquement.

7. Système de barres de serrage selon une des revendications précédentes, **caractérisé en ce que** les éléments de barre supérieures (11) et horizontalement voisins de la chaîne de barres supérieure (9) sont interconnectés par des bandes de liaison horizontales (12) et par des éléments de connexion verticaux et en particulier de façon à ce que les éléments de connexion des bandes de liaison (12) fonctionnent en même temps comme des éléments de fixation sans jeu avec la face supérieure (2) de la paroi de conteneur (1) et/ou **en ce que** les points centraux (22, 23) des arrondis convexes (20 et concaves (21) correspondant aux éléments de barres supérieures voisins (11) se superposent approximativement.

8. Système de barres de serrage selon une des revendications précédentes, **caractérisé en ce que** des éléments de barres (10, 11) horizontalement voisins de la chaîne de barres supérieure (9) et inférieure (8) sont interconnectés par des éléments de fixation verticaux.

9. Système de barres de serrage selon la revendication 8, **caractérisé en ce que** des éléments de barres (10, 11) verticalement voisins de la chaîne de barres supérieure (9) et de la chaîne de barres inférieure (8) sont interconnectés à leurs extrémités respectives à l'aide des éléments de fixation verticaux en forme de boulons ou de vis traversant des ouvertures ou des perçages (26) dans des éléments de barre (10, 11) et ancrés dans la paroi de conteneur (1).

10. Système de barres de serrage selon une ou plusieurs des revendications précédentes, **caractérisé en ce que** les éléments de barre (10) de la chaîne de barres inférieure (8) sont rendus étanches au gaz par rapport à la face supérieure (2) de la paroi de conteneur (1) et ceci en particulier par une matière étanche(19) déposée en forme d'anneau, élastique lors du montage et durcissant après montage et/ou **en ce qu'**au moins les éléments de barre (10) de la chaîne de barres inférieure (8) possèdent des ouvertures ou des perçages (27) séparés à travers lesquels la matière élastique (19) s'étend lors du montage sur la face supérieure (2) de la paroi de conteneur (1), créant après durcissement un lien sans jeu.

11. Système de barres de serrage selon la revendication 10, **caractérisé en ce que** les bords des arrondis concaves et/ou convexes (20, 21) des éléments de barres inférieures (10) possèdent des évidements (28) à travers lesquels la matière étanche(19) passe lors du montage sur la face supérieure (2) de la paroi de conteneur (1) et forme après durcissement une connexion sans jeu et/ou étanche au gaz.

12. Système de barres de serrage selon une des revendications précédentes, **caractérisé en ce que** tous ou presque tous les éléments de barres (10, 11) de la chaîne de barres inférieure (8) et de la chaîne de barres supérieure (9) sont essentiellement identiques.

13. Elément de barres destiné à un système de barres de serrage selon une des revendications précédentes, **caractérisé en ce que** la distance axiale (29) entre le point le plus bas de l'arrondi convexe (20) et le point le plus haut de l'arrondi concave (21) d'un élément de barre (10, 11) est d'une longueur effective (29) d'environ n/4 mètre, donc environ 78,54 cm +/-1%.

14. Elément de barres selon la revendication 13, **caractérisé en ce que** l'arrondi convexe (20) décrit un demi-cercle de 180° et **en ce que** l'arrondi concave (21) décrit un arrondi de moins d'un demi-cercle, en particulier une section de cercle allant d'environ 90° à environ 135° et/ou **en ce que** l'élément de barre (10, 11) possède dans la zone de l'arrondi concave (21) une largeur (30) légèrement réduite vers l'arrondi concave (21), c'est-à-dire que l'élément est légèrement pointu vers son extrémité formant un angle (25)par rapport à la normale situé entre 1° et 5° environ.

15. Elément de barres destiné à un système de barres de serrage selon la revendication 13 ou 14, caractérisé en ce quel'élément de barre (10, 11) possède dans la zone de l'arrondi convexe (20) une largeur (30) légèrement réduite, créant la place dans laquelle les extrémités de l'arrondi concave (21) de l'élément de barre (10, 11) horizontalement voisines s'enfoncent lors d'un pivotement avec un angle de pivotement (14).
